# EUROPÄISCHE PATENTANMELDUNG

(11) **EP 0 999 170 A1**
(43) Veröffentlichungstag der Anmeldung: **10.05.2000**
(21) Anmeldenummer: 99122170.6
(22) Anmeldetag: 05.11.1999
(51) Int. Cl.: B66C 1/44

(54) **Greifersystem**

(30) Priorität: 06.11.1998 DE 19851292
(71) Anmelder: Fibro GmbH, D-74189 Weinsberg (DE)
(72) Erfinder: Weiser, Kurt, 74831 Gundelsheim (DE); Walter, Michael, 74869 Schwarzach (DE); Massmann, Hans-Joachim, 74831 Gundelsheim (DE); Kratzer, Willi, 74842 Billigheim (DE)
(74) Vertreter: Manitz, Finsterwald & Partner

(57) **Zusammenfassung**

Es wird ein Greifersystem zur Handhabung von großvolumigen Teilen mit an einem Horizontalträger (1) geführten Spannarmen (2,3) beschrieben, die im Bereich ihrer freien Enden Spannflansche (4,5) aufweisen, wobei die Spann- oder Adapterflansche (4,5) drehbar gelagert und unabhängig von der jeweiligen Öffnungsweite der Spannarme (2,3) synchron drehbar sind.

## Beschreibung

Die Erfindung betrifft ein Greifersystem zur Handhabung von großvolumigen Teilen, insbesondere zur Handhabung von Motorblöcken, Zylinderköpfen, Getriebegehäusen u.dgl., mit zwei einander gegenüberliegend angeordneten, an einem Horizontalträger geführten und relativ zueinander mittels einer ersten Antriebseinheit verfahrbaren Spannarmen, die im Bereich ihrer freien Enden Spann- oder Adapterflansche, insbesondere zur Aufnahme von werkstückspezifischen Spannbacken aufweisen.

Greifersysteme dieser Art sind prinzipiell bekannt und werden beispielsweise bei der Automatisierung von Produktionslinien eingesetzt.

Aufgabe der vorliegenden Erfindung ist es, ein in besonders wirtschaftlicher Weise zu fertigendes und funktionssicheres Greifersystem zu schaffen, das die Handhabung großvolumiger, jedoch in ihren Abmessungen auch unterschiedlicher Teile bei gleichzeitiger Ermöglichung einer Wendefunktion gestattet.

Gelöst wird diese Aufgabe nach der Erfindung im wesentlichen dadurch, daß die Spannflansche an den Spannarmen drehbar gelagert und unabhängig von der jeweiligen Öffnungsweite der Spannarme synchron über eine gemeinsame, am Horizontalträger angeordnete zweite Antriebseinheit drehbar sind.

Besonders günstig ist es, die beiden benötigten Antriebseinheiten bezüglich der verfahrbaren Spannarme zumindest im wesentlichen mittig und beiderseits des Horizontalträgers anzuordnen, das Verfahren der Spannarme mittels einer Rechts-Links-Gewindespindel vorzunehmen und den Antrieb der Spannflansche über eine Keilwelle zu realisieren. Auf diese Weise werden kostengünstige und besonders robuste und damit auch zuverlässige Antriebssysteme geschaffen, die einerseits die erforderlichen Spannkräfte ermöglichen und andererseits die über die drehbaren Spannflansche realisierte Wendefunktion unabhängig von der jeweiligen Öffnungsweite der Spannarme stets mit gleicher Wirksamkeit und Effektivität erbringen, so daß das Greifersystem ohne Schwierigkeiten auch programmgesteuert betrieben werden kann.

Durch die mittige Positionierung der Antriebseinheiten und die Verwendung kostengünstiger Schneckengetriebe zum Antrieb einer Mitnahmewelle, insbesondere einer Keilwelle und einer Verschiebewelle, insbesondere einer Rechts-Links-Gewindespindel wird nicht nur eine wirtschaftliche sondern auch technisch vorteilhafte Anordnung mit symmetrischer Lastverteilung erreicht.

Besonders günstig ist es, die Gewindespindel mittig in einer Hohlwelle des zur Antriebseinheit gehörenden Schneckengetriebes aufzunehmen, da dadurch die axialen Reaktionskräfte durch die Spannung des jeweiligen Werkstückes mit den Greifarmen nicht auf das Getriebelager einwirken, sondern nur in der Spindel selbst wirksam werden.

Dabei ist es besonders vorteilhaft, wenn durch Verwendung einer Spindelsteigung mit Selbsthemmung eine sichere Erhaltung der Spannlage und Spannkraft auch bei Abschaltung oder Ausfall der Antriebsenergie erreicht wird. Es liegt demgemäß bei dieser Ausführungsform eine einfache und wirksame Spannkraftsicherung vor.

Weitere vorteilhafte Ausgestaltungen der Erfindung sind in den Unteransprächen angegeben und werden bei der Erläuterung des Ausführungsbeispiels anhand der Zeichnung beschrieben; in der Zeichnung zeigt:
- Fig. 1: eine schematische Darstellung des Greifersystems mit der Antriebseinheit zur Spannarmbetätigung über eine Rechts-Links-Gewindespindel,
- Fig. 2: eine schematische Darstellung der gegenüberliegende Seite des Greifersystems mit der Antriebseinheit zur Spannflanschdrehung über eine Verschiebe-Mitnahmewelle (z.B. Keilwelle) und Zahnriementrieb, und
- Fig. 3: eine stirnseitige Ansicht des Greifersystems mit Spannflansch-Antriebselementen und Spindelantrieb.

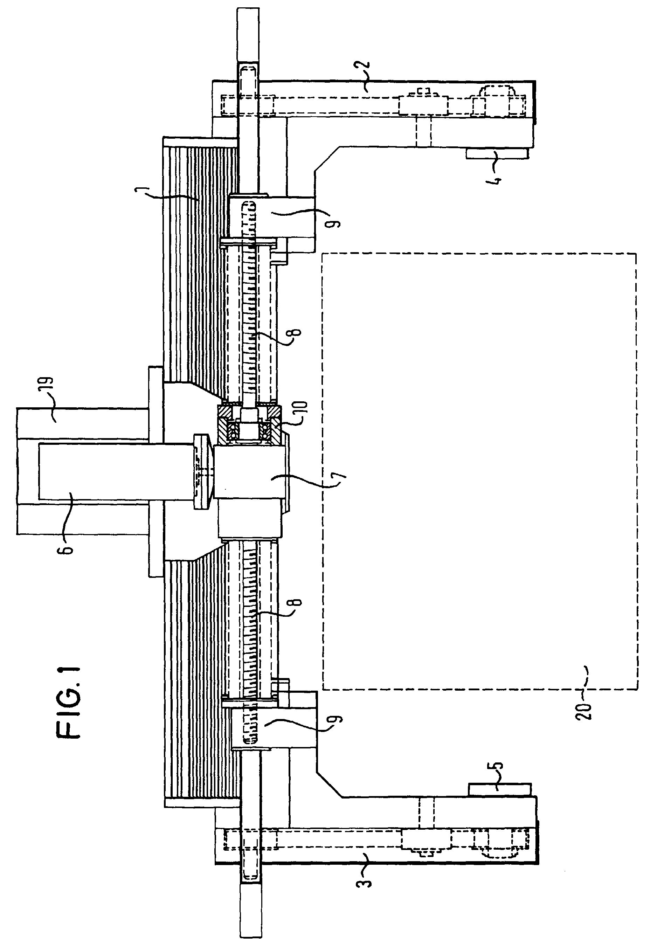

Fig. 1 zeigt ein Greifersystem umfassend einen als Profilteil ausgebildeten Horizontalträger 1 sowie zueinander gegenüberliegend angeordnete, am Horizontalträger 1 geführte Spannarme 2, 3, die im Bereich ihrer freien Enden mit drehbaren Spann- oder Adapterflanschen 4, 5 zur Aufnahme von werkstückspezifischen Spannbacken versehen sind.

Eine erste mittig und auf einer Seite des Horizontalträgers 1 angeordnete Antriebseinheit 6 treibt über ein Schneckengetriebe 7 eine Antriebsspindel 8 mit Rechts-Linksgewinde an, die endseitig in Führungsgewindeteile 9 der Spannarme 2, 3 eingreift, so daß in der einen Drehrichtung der Antriebseinheit 6 die Spannarme auseinandergefahren und in der anderen Drehrichtung zusammengefahren werden. Dadurch kann sich das Greifersystem dem jeweils aufzunehmenden Gegenstand, insbesondere unterschiedlich großen Motorblöcken, Getriebegehäusen u.dgl., anpassen.

Die Rechts-Links-Antriebsspindel 8 ist durch eine Hohlwelle des Schneckengetriebes 7 gesteckt und wird über dieses Schneckengetriebe angetrieben, wobei der damit realisierte Mittenantrieb dieser Gewindespindel zu dem technischen Vorteil führt, daß die axialen Reaktionskräfte durch die Halterung des Werkstückes nicht auf das Getriebelager, sondern nur in der Spindel selber wirken.

Um außergewöhnliche Kräfte, die durch Fehlspannung oder äußere Einwirkung beim Auffahren entstehen können, nicht auf das Getriebelager wirken zu lassen, sind einerseits oder beiderseits des Getriebes zusätzliche Lager 10 zur Aufnahme dieser Kräfte vorgesehen, die am Getriebegehäuse abgestützt werden.

Der Horizontalträger 1 ist mittig mit einem Vertikalträger 19 verbunden, der insbesondere Bestandteil eines Portalsystems ist. Zwischen dem Vertikalträger 19 und dem Horizontalträger 1 kann auch eine Rotations- oder Translationseinheit bei Bedarf vorgesehen sein.

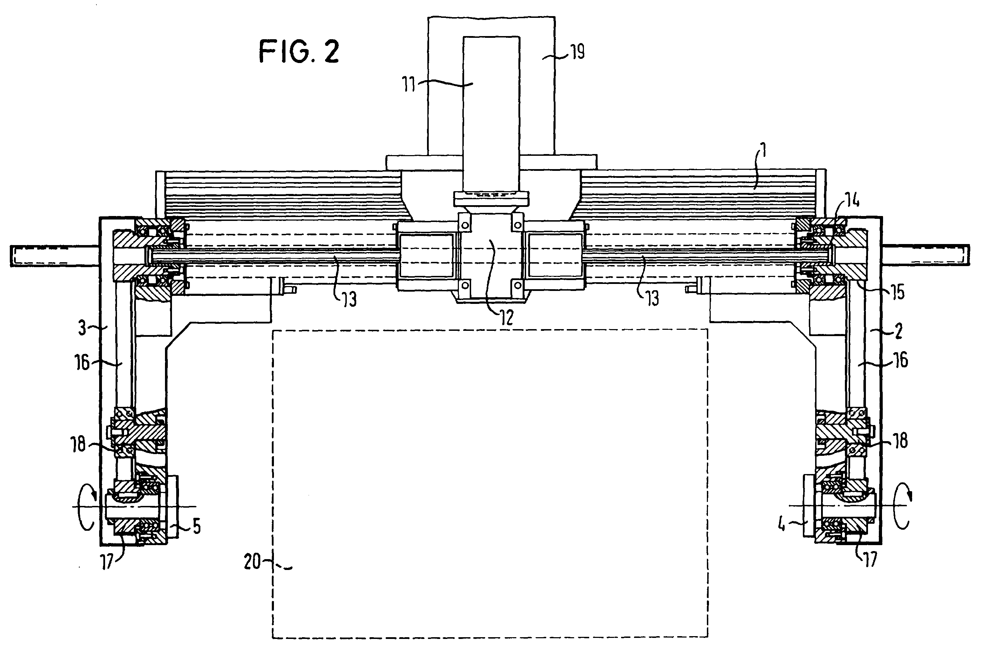

Fig. 2 zeigt das Greifersystem von der anderen Seite des Horizontalträgers 1, wo die zweite Antriebseinheit 11 mit zugeordnetem Schneckengetriebe 12 für eine vorzugsweise durchgehende Keilwelle 13 angeordnet ist. Diese Keilwelle 13 erstreckt sich durch in den Spannarmen 2, 3 drehbar gelagerte Übertragungsorgane 14 und versetzt diese Übertragungsorgane 14 unabhängig von dem jeweiligen gegenseitigen Abstand der Spannarme 2, 3 in Abhängigkeit von der Ansteuerung der zweiten Antriebseinheit 11 in Drehung. Diese Drehung wird über die außenliegende Verzahnung 15 dieser Übertragungsorgane 14 mittels eines Zahnriemens 16 zu den Spannflanschen 4, 5 übertragen, welche mit entsprechenden Zahnrädern 17 verkeilt sind. Diese besonders einfache und betriebssichere Antriebsart stellt auch sicher, daß der Aufnahmeraum zwischen den Spannarmen 2, 3 nicht beeinträchtigt wird und jeder zwischen den Spannarmen 2, 3 aufgenommene Gegenstand mittels der Spannflansche 4 und 5 über 360° geschwenkt und damit in der jeweils erforderlichen Weise abgesetzt werden kann.

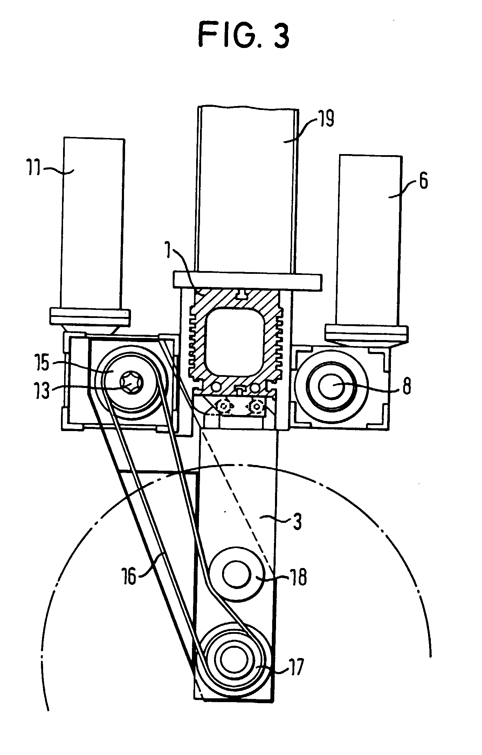

Die Stirnansicht nach Fig. 3 zeigt die beiden beiderseits des Horizontalträgers 1 angeordneten Antriebseinheiten 6, 11 mit den zugehörigen Getrieben und Führungen und verdeutlicht die ausgewogene Gesamtkonstruktion des Greifersystems mit Wendefunktion. Zu sehen ist dabei auch der Verlauf des zum Antrieb der Spannflansche vorgesehenen Zahnriemens 16, der über eine Spannrolle 18 spannbar ist. Die Gesamtanordnung ist trotz der erzielten Mehrfachfunktionen kompakt und im Hinblick auf die Spann- und Wendebewegungen frei programmierbar.

### Bezugszeichenliste

- 1: Horizontalträger
- 2: Spannarm
- 3: Spannarm
- 4: Spannflansch
- 5: Spannflansch
- 6: erste Antriebseinheit für Spannhub
- 7: Schneckengetriebe
- 8: Rechts-Links-Antriebsspindel
- 9: Führungsgewindeteil
- 10: Zusatz-Stützlager
- 11: zweite Antriebseinheit für Dreh- bzw. Wendebewegung
- 12: Schneckengetriebe
- 13: Keilwelle
- 14: Übertragungsorgan
- 15: Verzahnung
- 16: Zahnriemen
- 17: Zahnriemenrad
- 18: Spannrolle
- 19: Vertikalträger
- 20: Motorblock

## Patentansprüche

1. Greifersystem zur Handhabung von großvolumigen Teilen, insbesondere zur Handhabung von Motorblöcken, Zylinderköpfen, Getriebegehäusen u.dgl., mit zwei einander gegenüberliegend angeordneten, an einem Horizontalträger (1) geführten und relativ zueinander mittels einer ersten Antriebseinheit (6) verfahrbaren Spannarmen (2, 3), die im Bereich ihrer freien Enden Spann- oder Adapterflansche (4, 5), insbesondere zur Aufnahme von werkstückspezifischen Spannbacken aufweisen,
dadurch **gekennzeichnet,**
daß die Spann- oder Adapterflansche (4, 5) an den Spannarmen (2, 3) drehbar gelagert und unabhängig von der jeweiligen Öffnungsweite der Spannarme (2, 3) synchron über eine gemeinsame, am Horizontalträger (1) angeordnete zweite Antriebseinheit (11) drehbar sind.

2. Greifersystem nach Anspruch 1,
dadurch **gekennzeichnet,**
daß die beiden Antriebseinheiten (6, 11) bezüglich der verfahrbaren Spannarme (2, 3) zumindest im wesentlichen mittig und beiderseits des Horizontalträgers (1) angeordnet sind.

3. Greifersystem nach Anspruch 1 oder 2,
dadurch **gekennzeichnet,**
daß die erste Antriebseinheit (6) zum Verfahren der Spannarme (2, 3) mit einer Rechts-Links-Gewindespindel (8) gekuppelt ist, deren mit einem Rechts- bzw. Linksgewinde versehene Hälften mit ihren freien Endbereichen jeweils in ein spannarmfestes Führungsgewinde (9) eingreifen.

4. Greifersystem nach Anspruch 3
dadurch **gekennzeichnet,**
daß die Rechts-Links-Gewindespindel (8) mit selbsthemmender Steigung ausgeführt ist und damit die jeweilige Halteposition und Spannkraft auch bei Ausfall oder bei Abschalten der Antriebsenergie erhalten bleibt.

5. Greifersystem nach Anspruch 3 oder 4,
dadurch **gekennzeichnet,**
daß die Gewindespindel (8) mittig in einer Hohlwelle eines zur ersten Antriebseinheit (6) gehörenden Schneckengetriebes aufgenommen ist.

6. Greifersystem nach Anspruch 1,
dadurch **gekennzeichnet,**
daß ein- oder beidseitig der Hohlwellenaufnahme der Gewindespindel (8) jeweils ein am Getriebegehäuse abgestütztes Zusatzlager (10) vorgesehen ist.

7. Greifersystem nach einem oder mehreren der vorhergehenden Ansprüche,
dadurch **gekennzeichnet,**
daß die zweite Antriebseinheit (11) für die Spann- oder Adapterflansche (4, 5) mit einer sich über den Verfahrweg der Spannarme (2, 3) erstreckenden Keilwelle (13) gekuppelt ist, die in den beiden Spannarmen (2, 3) drehbar gelagerte und außerhalb der Spannanne (2, 3) eine Verzahnung (15) aufweisende Übertragungsorgane (14) kraftschlüssig und beweglich durchsetzt.

8. Greifersystem nach Anspruch 7,
dadurch **gekennzeichnet,**
daß die drehbar gelagerten Spann- oder Adapterflansche (4, 5) an der Spannarmaußenseite über ein Zahnrad (17) antreibbar sind, das über einen Zahnriemen (16) mit dem zugehörigen, von der Keilwelle (13) durchsetzten Übertragungsorgan (14) verbunden ist.

9. Greifersystem nach einem der vorhergehenden Ansprüche,
dadurch **gekennzeichnet,**
daß die zweite Antriebseinheit (11) die Keilwelle (13) über ein Schneckengetriebe antreibt.

10. Greifersystem nach einem der vorhergehenden Ansprüche,
dadurch **gekennzeichnet,**
daß der Horizontalträger (1) aus einem Aluminiumprofilkörper besteht und mittig mit einem verfahrbaren Vertikalträger (19) verbunden ist.

11. Greifersystem nach einem der vorhergehenden Ansprüche,
dadurch **gekennzeichnet,**
daß zwischen dem Vertikalträger (19) und dem Horizontalträger (1) eine Dreh- und/oder Translationseinheit zum zusätzlichen Verfahren oder Positionieren des jeweiligen Werkstückes angeordnet ist.

12. Greifersystem nach einem oder mehreren der vorhergehenden Anspräche,
dadurch **gekennzeichnet,**
daß die aufeinanderfolgenden Spann- und Schwenkvorgänge programmgesteuert sind.
